# FASCICULE DE BREVET EUROPEEN

(11) **EP 1 075 224 B1**
(45) Date de publication et mention de la délivrance du brevet: **05.03.2003**
(21) Numéro de dépôt: 99916956.8
(22) Date de dépôt: 29.04.1999
(51) Int. Cl.: A61B 17/70

(54) **SYSTEME D'OSTEOSYNTHESE RACHIDIENNE AVEC MOYENS DE SERRAGE NOTAMMENT POUR UNE FIXATION ANTERIEURE**
WIRBELSÄULEN-OSTEOSYNTHESESYSTEM MIT SPANNVORRICHTUNG, INSBESONDERE ZUR VORDEREN FIXIERUNG
BACKBONE OSTEOSYNTHESIS SYSTEM WITH CLAMPING MEANS IN PARTICULAR FOR ANTERIOR FIXING

(30) Priorité: 29.04.1998 FR 9805387; 09.10.1998 FR 9812662
(43) Date de publication de la demande: 14.02.2001
(73) Titulaire: Dimso Distribution Medicale du Sud Ouest, 33610 Cestas (FR)
(72) Inventeur: ASSAKER, Richard, B-7540 Kain (BE); CONCHY, Frédéric, F-33650 Saint-Médard-d'Eyrans (FR); LE COUEDIC, Régis, F-33610 Cestas (FR)
(74) Mandataire: Le Forestier, Eric
(86) Numéro de dépôt international: FR9901020
(87) Numéro de publication internationale: WO99055247

(56) Documents cités:
- DE-A- 19 534 136
- DE-U- 9 412 744
- DE-U- 29 712 697
- US-A- 4 987 892
- US-A- 5 613 968

## Description

L'invention concerne les systèmes d'ostéosynthèse rachidienne notamment de fixation antérieure.

On connaît d'après le document FR-2 731 344 un système d'ostéosynthèse rachidienne comprenant une tige, une vis vertébrale présentant un corps à ancrer dans une vertèbre et une tête sous la forme d'une tige filetée. Le système comprend un connecteur à deux branches aptes à être engagées sur la tête de vis. Un écrou de serrage peut être enfilé sur la tête de vis pour serrer les deux branches entre la base de la tête de vis et l'écrou. La tige engagée entre les branches est ainsi serrée et immobilisée.

Ce système présente de nombreux avantages. Comportant peu de pièces et facile à installer, il permet une mise en place rapide lors d'une intervention chirurgicale. Toutefois, l'écrou a un profil externe hexagonal à six pans nécessaire pour permettre sa manoeuvre et son serrage au moyen d'une clé femelle. Or, un tel profil génère de nombreuses arêtes vives et coins saillants susceptibles de blesser les tissus corporels venant en contact avec l'écrou. De même, on peut difficilement éviter qu'une partie d'extrémité filetée de la tête de vis fasse saillie hors de l'écrou à l'issue du serrage. Or, ce filet constitue lui aussi des arêtes saillantes nuisibles pour le corps.

Un but de l'invention est de réduire le nombre de parties saillantes sur le système une fois celui-ci installé.

En vue de la réalisation de ce but, on prévoit selon l'invention un système d'ostéosynthèse rachidienne, notamment de fixation antérieure, comprenant :
- un élément de liaison allongé ;
- une vis vertébrale présentant une tète filetée ;
- un connecteur comportant deux branches aptes à être enfilées sur la vis et à serrer entre elles l'élément de liaison ; et
- un organe de serrage fileté apte à coopérer avec la tête pour serrer les branches,
dans lequel la tête présente un orifice fileté, l'organe de serrage comprenant une tige filetée apte à venir en prise avec l'orifice.

Ainsi, le filet de la vis vertébrale s'étend dans un orifice et l'orifice fileté de la vis est obturé par l'organe de serrage à l'issue de la mise en place. Aucun contact du corps du patient n'est donc plus possible avec le filet de la vis vertébrale. De plus, l'organe de serrage étant un organe mâle, on peut y ménager une empreinte de manoeuvre à profil polygonal destinée à coopérer avec un instrument mâle pour la manoeuvre de l'organe de serrage et donc se dispenser des arêtes externes. On réduit ainsi également le nombre de parties saillantes sur l'organe de serrage. En outre, le connecteur est enfilé sur la tête de la vis vertébrale sans interférer avec un filet de celle-ci. Son calage est donc meilleur, même avant serrage par l'organe de serrage. On peut donc prépositionner le connecteur sur la vis vertébrale de façon fiable.

Avantageusement, l'organe de serrage présente une empreinte à profil polygonal suivant un axe longitudinal de l'organe.

Avantageusement, l'organe de serrage comprend une tête présentant une face inférieure sphérique convexe, une desdites branches du connecteur présentant une face supérieure sphérique concave apte à être en contact avec la face convexe lors du serrage des branches.

On permet ainsi un réglage de la position angulaire relative du connecteur et de la vis vertébrale rigidement fixée à l'organe de serrage.

Avantageusement, l'orifice de la vis vertébrale présente une empreinte à profil polygonal suivant un axe longitudinal de la vis.

Cette empreinte, permettant la manoeuvre de la vis vertébrale, réduit elle aussi le nombre de parties saillantes.

Avantageusement, l'empreinte s'étend dans le filet de l'orifice.

Ainsi, en dépit de la présence du filet et de l'empreinte, on peut donner à la tête de la vis vertébrale, et donc au système, des dimensions réduites.

Avantageusement, le profil de l'empreinte de la vis vertébrale et le profil de l'empreinte de l'organe de serrage ont même forme et mêmes dimensions.

On peut donc manoeuvrer la vis vertébrale et l'organe de serrage au moyen d'un même instrument mâle, ce qui réduit l'équipement nécessaire à l'intervention chirurgicale et réduit le risque d'erreur dans le choix des instruments.

Avantageusement, la tête de la vis vertébrale présente une face latérale d'extrémité externe lisse.

Avantageusement, le système comprend une deuxième vis vertébrale, l'une desdites branches présentant un prolongement apte à être engagé sur la deuxième vis.

Ainsi, la fixation du connecteur à la vertèbre au moyen des deux vis permet d'assurer un positionnement précis, stable et sûr du connecteur et donc de l'élément de liaison.

Avantageusement, le système comprend un deuxième élément de liaison allongé, le connecteur étant apte à être fixé simultanément aux deux éléments de liaison.

Ainsi, la présence des deux éléments de liaison confère au système une très grande rigidité, sans compliquer son assemblage, sans accroître le volume de ses différentes pièces (ce qui le rend compatible avec un montage par voie endoscopique), et tout en conservant le cas échéant la possibilité de réglage de la position angulaire du connecteur par rapport au premier élément de liaison. Le système selon l'invention ne requiert pas un cintrage identique sur les deux éléments de liaison. De plus, le nombre de connecteurs peut rester peu élevé.

Le système selon l'invention peut être mis en place par voie endoscopique et est adapté à être fixé en position antérieure.

D'autres caractéristiques et avantages de l'invention apparaîtront encore dans la description suivante de deux modes préférés de réalisation donnés à titre d'exemples non limitatifs. Aux dessins annexés :
- la figure 1 est une vue en perspective du système selon un premier mode de réalisation de l'invention ;
- la figure 2 est une vue partielle en perspective éclatée du système de la figure 1 ;
- les figures 3 et 4 sont deux vues en perspective, respectivement de dessus et de dessous, montrant un des connecteurs du système de la figure 1 ;
- la figure 5 est une vue, moitié en élévation et moitié en coupe axiale, d'une bague du système de la figure 1 ;
- la figure 6 est une vue, pour partie de dessus et pour partie en coupe, du connecteur de la figure 3 recevant la tige ;
- la figure 7 est une vue partielle en perspective montrant la tête de la vis principale ;
- la figure 8 montre le système de la figure 1 fixé sur des vertèbres ;
- la figure 9 est une vue en perspective du système selon un deuxième mode de réalisation de l'invention ;
- la figure 10 est une vue partielle en perspective éclatée du système de la figure 9 ;
- les figures 11 et 12 sont deux vues en perspective, respectivement de dessus et de dessous, montrant un des connecteurs du système de la figure 9 ; et
- la figure 13 montre le système de la figure 9 fixé sur des vertèbres.

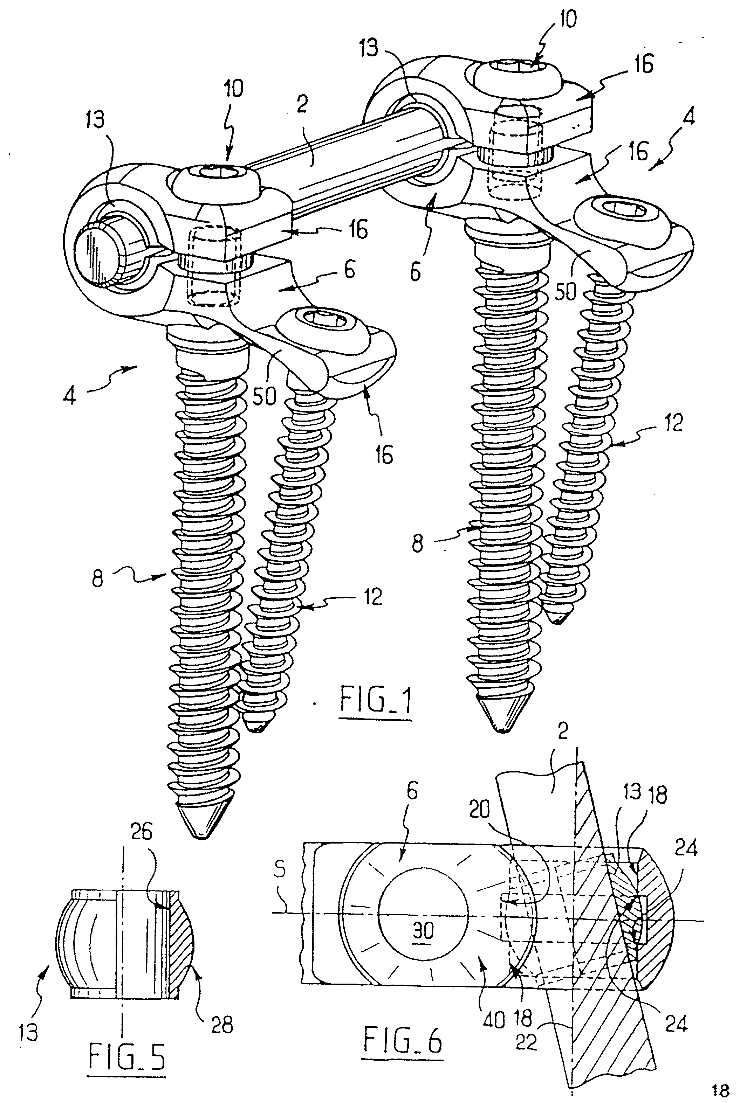

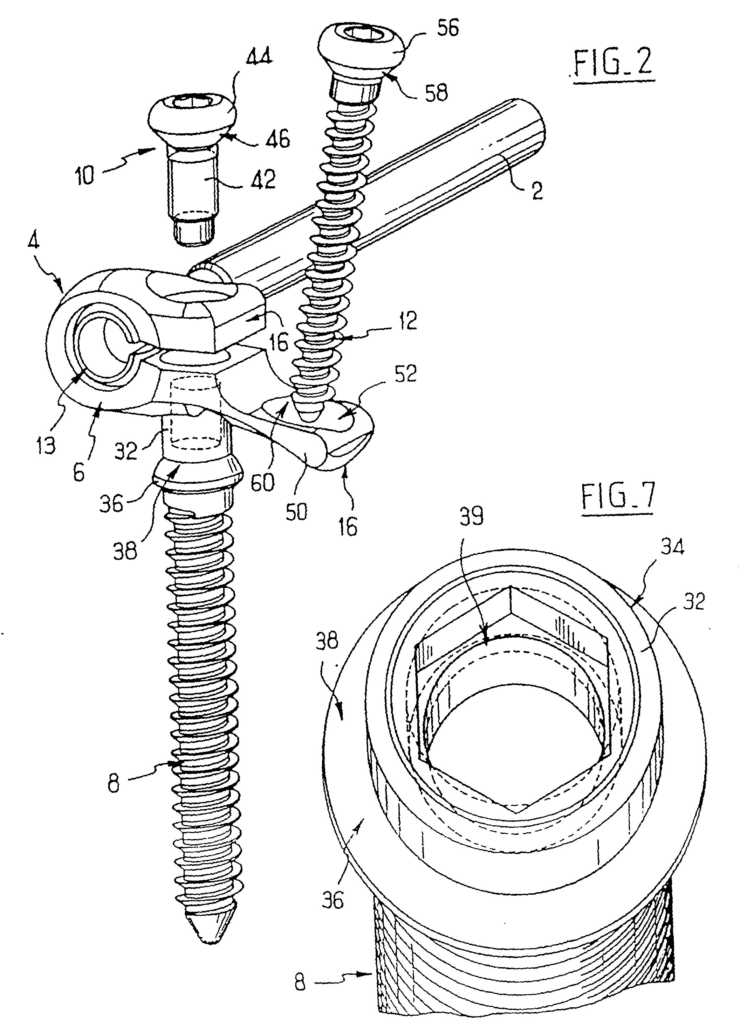

En référence aux figures 1 à 8, le système selon l'invention comprend dans le premier mode de réalisation une tige de liaison allongée 2 à section circulaire et plusieurs sous-ensembles 4 à connecteur aptes à être fixés à celle-ci. Chacun de ces sous-ensembles, dont deux sont visibles sur la figure 1 et un seul est visible sur la figure 2, comprend un connecteur 6, une première vis vertébrale ou vis principale 8, une vis de serrage 10, une deuxième vis vertébrale ou vis secondaire 12, et une bague 13.

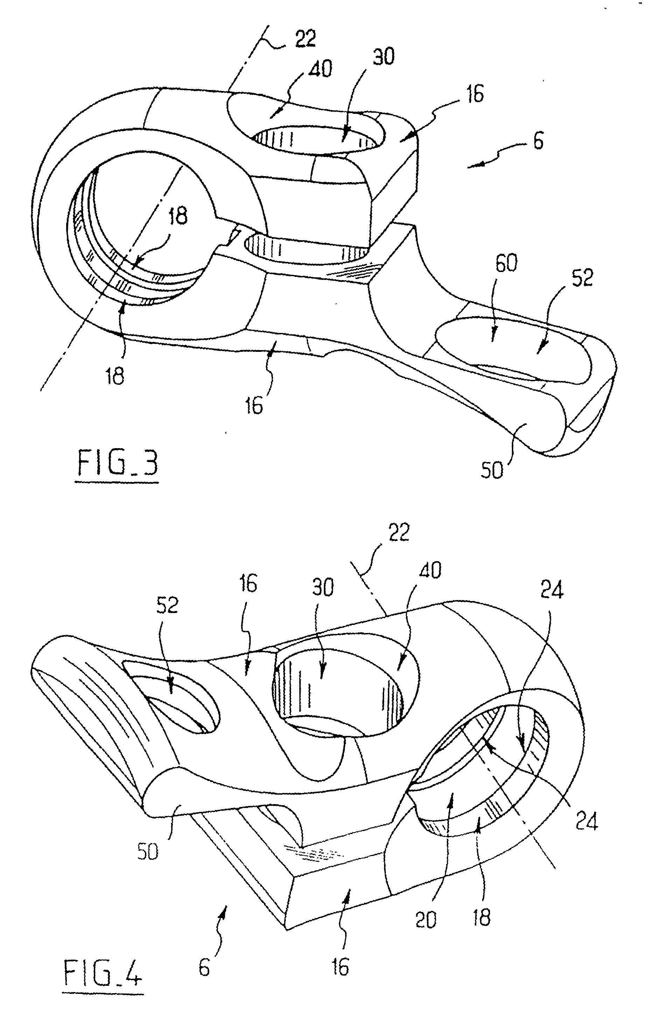

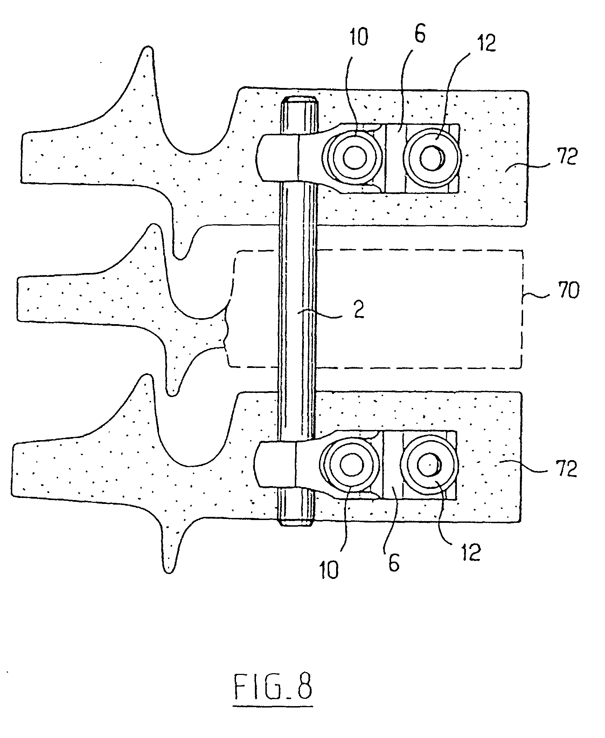

En référence aux figures 3 et 4, le connecteur 6 comporte deux branches 16 s'étendant en regard et à distance l'une de l'autre en donnant au connecteur un profil général en "U". Le connecteur 6 comporte un plan de symétrie S perpendiculaire à la largeur des branches 16 et parallèle à leur longueur. En référence à la figure 6, au niveau de la naissance des branches 16, le connecteur présente deux faces internes cylindriques coaxiales 18, 20 d'axe 22 perpendiculaire au plan « S » et de rayons différents, la face 20 de plus grand rayon est en deux parties disjointes et s'étend de part et d'autre de la face 18 de plus petit rayon, laquelle est traversée par le plan « S ». Les deux faces 18, 20 forment à leurs jonctions deux arêtes circulaires 24 d'axe 22.

La bague 13 présente une face interne cylindrique 26 et une face externe sphérique 28 coaxiales. La face interne cylindrique 26 a un rayon environ égal à celui de la tige 2 de sorte que la bague 13, fendue sur un côté suivant son axe, peut être reçue par ajustement glissant sur la tige. Par ailleurs, la bague 13 peut être logée entre les branches 16 en regard des faces cylindriques 18, 20. La face externe sphérique 28 de la bague a un rayon adapté pour que dans cette position, les arêtes 24 du connecteur 6 soient en contact linéaire avec la face externe sphérique 28 de la bague 13 et lui servent d'appuis. Dans cette position, avant serrage des branches 16, la position angulaire de la tige 2 enfilée dans la bague 13 peut être réglée dans deux plans perpendiculaires entre eux sur une amplitude par exemple de 15° de part et d'autre d'une position moyenne de la tige dans laquelle la tige est perpendiculaire au plan « S ».

Les branches 16 présentent deux évidements cylindriques lisses respectifs qui sont en l'espèce des orifices traversants 30 s'étendant coaxialement en regard l'un de l'autre. La vis principale 8 est une vis vertébrale bicorticale et présente un corps fileté à cette fin de façon connue en soi. Elle présente une tête 32 ayant une face externe cylindrique lisse 34. A la jonction entre la tête et le corps, la vis comporte une collerette annulaire 36 ayant une face inférieure plane perpendiculaire à un axe longitudinal de la vis et une face supérieure tronconique 38 avec la section la plus étroite du tronc de cône situé du côté de la tête 32 de la vis. La tête 32 présente un orifice fileté 39 coaxial au corps de la vis et, ménagée dans la face filetée de l'orifice 39, une forme non circulaire telle qu'une empreinte hexagonale à six pans. La vis de serrage 10 comporte un corps fileté 42 apte à former une liaison vis-écrou avec cet orifice 39, et une tête de vis 44 dans laquelle est formée une empreinte hexagonale à six pans. La tête 44 a une face externe inférieure sphérique convexe 46 dont la section la plus étroite est située vers la pointe de la vis.

L'une des branches 16, que pour la clarté nous appellerons ici branche inférieure, présente un prolongement 50 s'étendant en direction opposée aux faces cylindriques 18, 20 du connecteur. Il s'agit ici de la branche destinée à être adjacente à la vertèbre. Les deux branches 16 sont aptes à être enfilées simultanément sur la tête 32 de la vis principale 8 introduite à partir de la branche inférieure contre laquelle la face supérieure 38 de la collerette 36 vient en butée. On introduit ensuite la vis de serrage 10 dans la tête 32 de la vis principale 8 à partir de la branche supérieure 16. Le serrage de la vis 10 dans la tête 32 de la vis principale 8 provoque le rapprochement des branches 16 l'une de l'autre et le blocage par friction de la tige 2 dans la position choisie par rapport au connecteur 6.

L'orifice 30 de la branche inférieure 16 a un bord inférieur, opposé à la branche supérieure et destiné à être du côté de la vertèbre, présentant une empreinte sphérique concave 40 destinée à venir en contact avec la face supérieure 38 de la collerette 36 pour réaliser par friction un blocage à rotation du connecteur 6 par rapport à l'axe de la vis principale 8. L'orifice 30 de la branche supérieure 16 a un bord supérieur, opposé à la branche inférieure et destiné à être opposé à la vertèbre, présentant une empreinte sphérique concave 40 destinée à venir en contact avec la face inférieure sphérique convexe 46 de la tête 44 de la vis de serrage 10 et permettant de fixer celle-ci ainsi que la vis principale 8 en réglant l'orientation angulaire de la vis principale 8 par rapport au connecteur.

Le prolongement 50 présente un évidement sous la forme d'un orifice traversant 52. La branche inférieure 16 est courbée au niveau du prolongement 50 dans une direction opposée à la branche supérieure 16 de sorte que les axes des orifices 30 et 52 qu'elle porte ne sont pas tout à fait parallèles. La vis secondaire 12 est une vis vertébrale, ici monocorticale, présentant un corps fileté et une tête 56 à face inférieure sphérique convexe 58 dont la section la plus étroite se trouve du côté du corps. Sa tête présente une empreinte à six pans. L'orifice 52 du prolongement a un bord supérieur, orienté du côté de l'autre branche 16 et destiné à être opposé à la vertèbre, présentant une empreinte sphérique concave 60 destinée à venir en contact avec la face inférieure sphérique convexe 58 de la tête 56 de la vis secondaire 12 en permettant de régler l'orientation angulaire de cette vis par rapport au connecteur 6.

On retrouvera dans les documents FR-2 731 344 et WO-96/27340 homologues précités certaines des caractéristiques du connecteur 6 qui n'ont pas été développées ici en grand détail.

La branche inférieure 16 peut être cintrée pour accentuer ou réduire sa courbure en vue de mieux s'adapter à la forme de la partie antérieure de sa vertèbre de destination. Une fois cintrée, cette branche 16 n'est plus sollicitée en flexion puisqu'elle est fixée à la vertèbre par deux vis 8, 12 sur sa longueur. Les deux vis principale 8 et secondaire 12 sont autotaraudeuses et comportent des filetages à os.

Dans une variante de réalisation, la vis principale 8 ne présente pas une empreinte à six pans dans son orifice fileté 39, mais la collerette 36 a une forme hexagonale ou présente deux méplats parallèles et diamétralement opposés l'un à l'autre pouvant .coopérer avec une clé de serrage pour mettre cette vis 8 en rotation par rapport au connecteur 6.

Dans le présent exemple, le connecteur 6 est d'une seule pièce. Les différentes parties du système sont en métal biocompatible.

La mise en place d'un tel dispositif s'effectue de la manière suivante en référence à la figure 8 : après exposition de la vertèbre affectée 70 et des deux vertèbres adjacentes 72, on réalise une corporectomie tout en préservant, quand cela est possible, les plateaux respectifs de celles-ci. Pour chaque sous-ensemble, on réalise un avant-trou sur le côté latéral de la vertèbre 72 associée à égale distance des plateaux supérieur et inférieur, ainsi qu'à la limite du quart le plus postérieur du corps vertébral. On insère ensuite la vis principale 8 dans cet avant-trou jusqu'à la collerette d'arrêt 36. le connecteur 6 est ensuite positionné sur ladite vis principale 8, bloqué en translation par la portée conique 38 de ladite vis principale 8 venant s'adapter à l'empreinte 40 du connecteur 6. On repère l'adaptation du connecteur 6 sur la vertèbre, que l'on peut ajuster par le retrait dudit connecteur pour cintrer la branche inférieure 16 qui est sa partie la plus antérieure.

On vient ensuite visser la vis secondaire 12 par rapport à la vis principale 8 dans le second orifice 52 de la branche inférieure 16 jusqu'au contact du logement sphérique 60 du prolongement prévu à cet effet avec la partie sphérique 58 de ladite vis secondaire 12. Il est souhaitable de positionner le connecteur 6 le plus parallèle possible aux plateaux.

Après avoir instrumenté les deux vertèbres adjacentes 72, on positionne la tige 2 dans les bagues des connecteurs 6 et on règle sa position angulaire sur chaque sous-ensemble. Le serrage final s'opère grâce à la vis de serrage 10 qui s'insère dans la vis principale 8 et comprime ainsi le connecteur 6 pour serrer la tige.

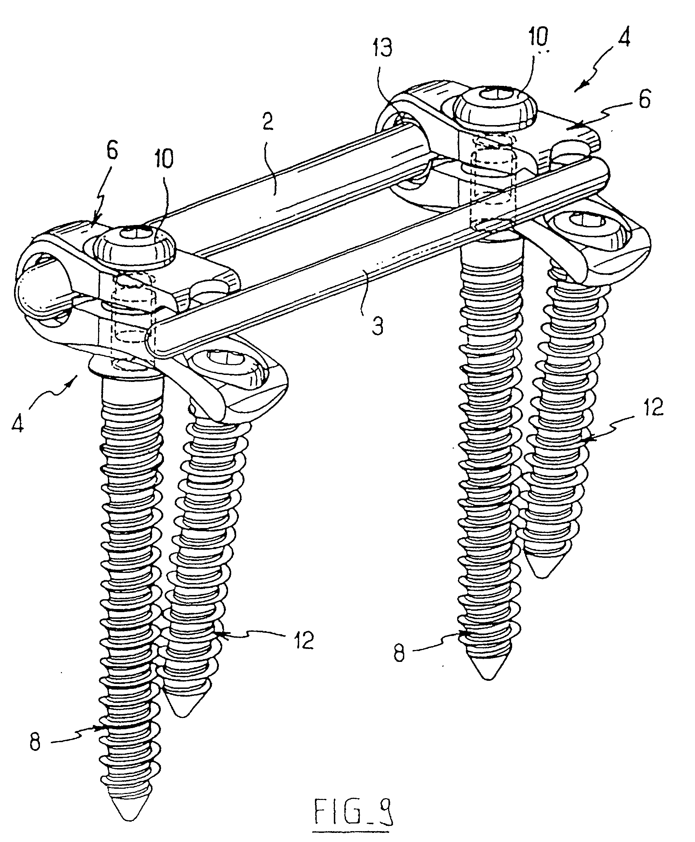

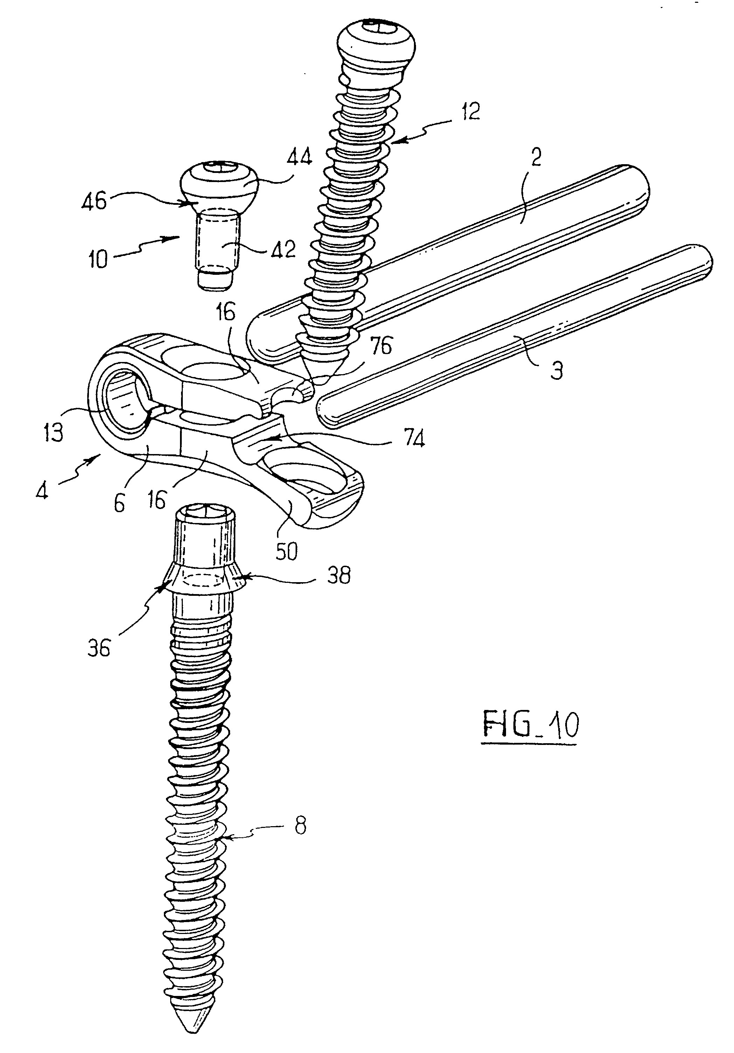

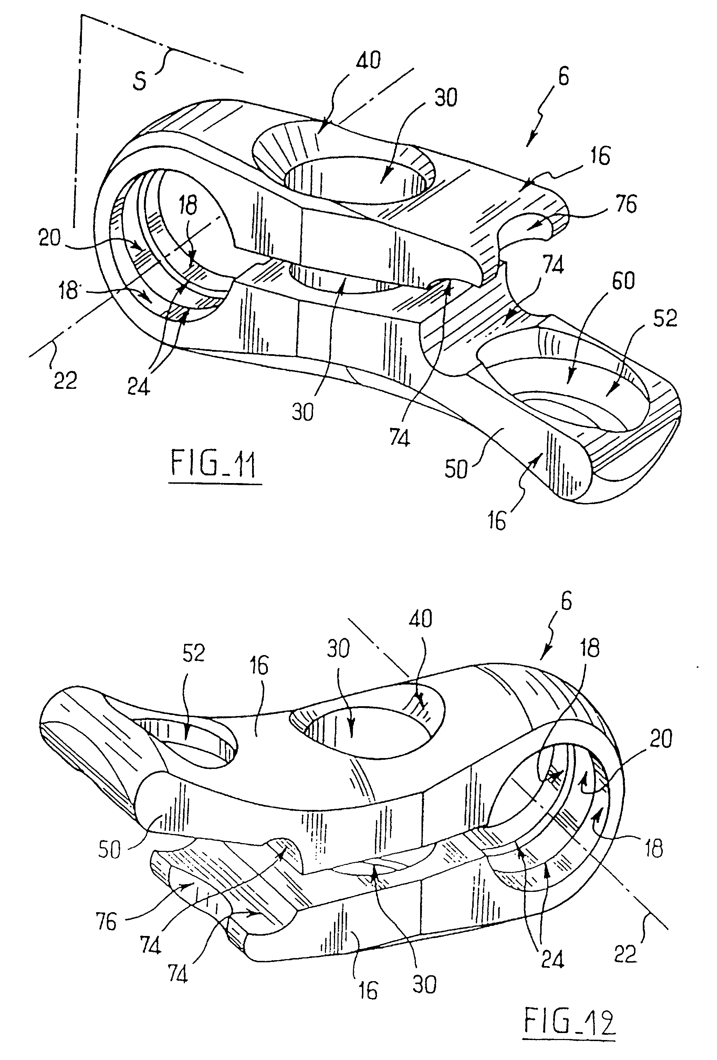

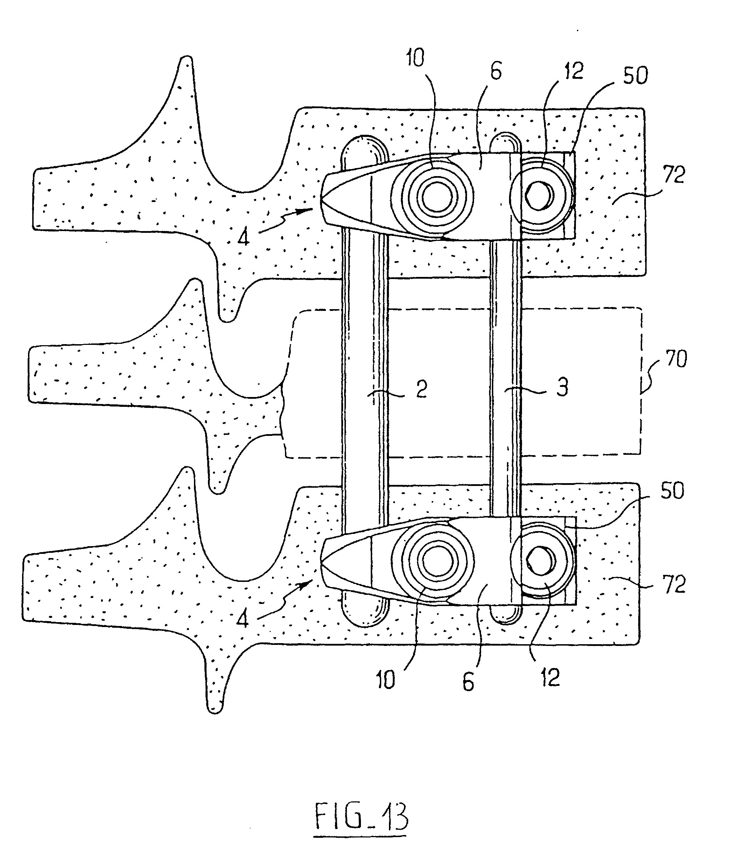

Dans le deuxième mode de réalisation illustré aux figures 9 à 13, le système est très proche de celui du premier mode. Il diffère cependant dans la présence d'une deuxième tige de liaison allongée 3 ou tige secondaire à section circulaire, et par l'adaptation du connecteur 6 à la réception de cette deuxième tige. La bague 13 est reçue sur la première tige ou tige principale 2.

Les deux tiges de liaison 2, 3 ont chacune une forme rectiligne profilée à profil ici circulaire. La tige secondaire 3 a une section, transversalement à son axe longitudinal, de diamètre inférieur à celui de la tige principale 2. La tige principale 2 aura par exemple un diamètre de 6 mm. Par exemple, le diamètre de la tige secondaire 3 sera compris entre 30% et 80% du diamètre de la tige principale 2. Ce faible diamètre permet au chirurgien de choisir la courbure de la tige secondaire 3 qui correspond à celle de l'étage du rachis instrumenté. En revanche, puisque les bagues 13 permettent une angulation relative entre les deux connecteurs 6, la tige principale 2 n'a pas besoin d'être cintrée. Elle peut donc avoir un diamètre important pour être très robuste.

Les branches 16 du connecteur présentent des empreintes cylindriques ou mors 74 respectives, ménagées dans les faces des branches en regard l'une de l'autre. Les empreintes 74 s'étendent en regard l'une de l'autre et ont des axes parallèles entre eux, et perpendiculaires au plan de symétrie S.

Sur la branche supérieure 16, l'empreinte 74 s'étend à une extrémité libre de la branche de sorte que l'orifice 30 est interposé entre les faces 18, 20 d'une part, et l'empreinte 74, d'autre part. Sur la branche inférieure 16, l'empreinte 74 s'étend entre les deux orifices 30 et 52, à la naissance du prolongement 50. Elle est contiguë à l'orifice 52 de sorte qu'elle mord sur son bord 60.

La tige secondaire 3 est destinée à être reçue dans l'empreinte 74 de la branche inférieure 16 dans une position angulaire unique par rapport au connecteur, perpendiculaire au plan de symétrie S. Lorsqu'on serre les deux branches 16 en direction l'une de l'autre, l'empreinte 74 de la branche supérieure vient en contact avec la tige secondaire 3 qui se trouve ainsi en contact surfacique avec chacune des deux empreintes qui réalisent un blocage à friction de la tige secondaire 3 par rapport au connecteur 6, lesquels sont ainsi rigidement fixés l'un à l'autre.

La tige secondaire 3 est placée dans l'empreinte 74 de la branche inférieure après que la vis secondaire 12 a été introduite dans l'orifice 52. La position de l'empreinte 74 de la branche inférieure fait en sorte que la tige secondaire 3 s'étend alors dans le trajet de la tête de la vis secondaire 12 pour son désengagement du connecteur et sa sortie de l'orifice 52. Par conséquent, une fois la tige secondaire 3 fixée au connecteur, la vis secondaire 12 ne peut plus être séparée du connecteur.

La branche supérieure 16 du connecteur présente à son extrémité libre une encoche 76 qui mord sur l'empreinte 74 à laquelle elle est contiguë et facilite la manoeuvre au moyen d'un instrument de la vis secondaire 12 en dépit de l'encombrement de la branche supérieure.

La mise en place du système selon le deuxième mode est similaire à celle du système du premier mode. La mise en place des vis principale 8 et secondaire 12 est inchangée.

Après avoir instrumenté les deux vertèbres adjacentes 72, on positionne la tige principale 2 dans les bagues 13 des connecteurs 6 et on règle la position angulaire de chaque sous-ensemble 4 par rapport à cette tige 2. On introduit ensuite la tige secondaire 3 dans les empreintes 74 des connecteurs 6 après l'avoir préalablement cintrée manuellement pour obtenir la courbure requise à l'étage rachidien correspondant. En cas de défaut, cette tige 3 peut être ôtée pour corriger sa courbure puis replacée. La figure 9 montre le système avant serrage des branches. Le serrage final s'opère grâce à la vis de serrage 10 qui s'insère dans la vis principale 8 et comprime ainsi le connecteur 6 pour serrer ses deux branches 16 l'une vers l'autre. Durant ce serrage, l'effort de serrage est dirigé d'abord sur la tige principale 2 via la bague 13, jusqu'à ce que l'empreinte 74 de la branche supérieure vienne en contact avec la tige secondaire 3. Dès lors, l'effort de serrage est réparti sur les deux tiges 2, 3. Ainsi, la réaction au niveau du couple vis principale 8/vis de serrage 10 est sensiblement coaxiale à celles-ci.

Lorsque le système est en place, les connecteurs 6, au moins au nombre de deux, sont rigidement et simultanément fixés chacun aux mêmes tiges principale et tige secondaire.

Dans chacun de ces modes de réalisation, on pourra indépendamment de la présence du prolongement 50 et de la deuxième vis 12, mettre en oeuvre les caractéristiques relatives à l'association de la première vis 8 avec la vis de serrage 10.

Bien que cela soit moins avantageux, la branche prolongée pourra être celle destinée à être la plus distante de la vertèbre.

On pourra mettre en oeuvre les caractéristiques relatives à la présence des deux vis vertébrales sur le connecteur indépendamment de celles relatives à la présence des tiges principale et secondaire, et réciproquement.

## Revendications

1. Système d'ostéosynthèse rachidienne, notamment de fixation antérieure, comprenant :
- un élément de liaison allongé (2) ;
- une vis vertébrale (8) présentant une tête filetée (32) ;
- un connecteur (6) comportant deux branches (16) aptes à être enfilées sur la vis (8) et à serrer entre elles l'élément de liaison (2) ; et
- un organe de serrage fileté (10) apte à coopérer avec la tète (32) pour serrer les branches (16),
**caractérisé en ce que** la tête (32) présente un orifice fileté (39), l'organe de serrage (10) comprenant une tige filetée (42) apte à venir en prise avec l'orifice (39).

2. Système selon la revendication 1, **caractérisé en ce que** l'organe de serrage (10) présente une empreinte à profil polygonal suivant un axe longitudinal de l'organe.

3. Système selon la revendication 1 ou 2, **caractérisé en ce que** l'organe de serrage (10) comprend une tête (44) présentant une face inférieure sphérique convexe (46), une desdites branches (16) du connecteur présentant une face supérieure sphérique concave (40) apte à être en contact avec la face convexe (46) lors du serrage des branches.

4. Système selon l'une quelconque des revendications 1 à 3, **caractérisé en ce que** l'orifice (39) de la vis vertébrale (8) présente une empreinte à profil polygonal suivant un axe longitudinal de la vis.

5. Système selon la revendication 4, **caractérisé en ce que** l'empreinte s'étend dans le filet de l'orifice (39).

6. Système selon la revendication 2 et la revendication 4, **caractérisé en ce que** le profil de l'empreinte de la vis vertébrale (8) et le profil de l'empreinte de l'organe de serrage (10) ont même forme et mêmes dimensions.

7. Système selon l'une quelconque des revendications 1 à 6, **caractérisé en ce que** la tête (32) de la vis vertébrale (8) présente une face latérale d'extrémité externe lisse (34).

8. Système selon l'une quelconque des revendications 1 à 7, **caractérisé en ce qu'**il comprend une deuxième vis vertébrale (18), l'une desdites branches (16) présentant un prolongement (50) apte à être engagé sur la deuxième vis.

9. Système selon l'une, quelconque des revendications 1 à 8, **caractérisé en ce qu'**il comprend un deuxième élément de liaison allongé (3), le connecteur (6) étant apte à être fixé simultanément aux deux éléments de liaison (2, 3).

## Claims

1. Spinal osteosynthesis system, in particular for anterior fixation, comprising:
- an elongate connection element (2);
- a vertebral screw (8) having a threaded head (32);
- a connector (6) including two branches (16) which can be engaged on the screw (8) and can clamp the connection element (2) between them; and
- a threaded clamping member (10) which can cooperate with the head (32) in order to clamp the branches (16),
**characterized in that** the head (32) has a threaded orifice (39), the clamping member (10) comprising a threaded rod (42) which can engage with the orifice (39).

2. System according to Claim 1, **characterized in that** the clamping member (10) has a recess of polygonal profile along a longitudinal axis of the member.

3. System according to Claim 1 or 2, **characterized in that** the clamping member (10) comprises a head (44) having a spherical and convex lower face (46), one of the said branches (16) of the connector having a spherical and concave upper face (40) which can be in contact with the convex face (46) upon clamping of the branches.

4. System according to any one of Claims 1 to 3, **characterized in that** the orifice (39) of the vertebral screw (8) has a recess of polygonal profile along a longitudinal axis of the screw.

5. System according to Claim 4, **characterized in that** the recess extends in the thread of the orifice (39).

6. System according to Claim 2 and Claim 4, **characterized in that** the profile of the recess of the vertebral screw (8) and the profile of the recess of the clamping member (10) have the same shape and the same dimensions.

7. System according to any one of Claims 1 to 6, **characterized in that** the head (32) of the vertebral screw (8) has a lateral face with a smooth outer end (34).

8. System according to any one of Claims 1 to 7, **characterized in that** it comprises a second vertebral screw (18), one of the said branches (16) having an extension (50) which can be engaged on the second screw.

9. System according to any one of Claims 1 to 8, **characterized in that** it comprises a second elongate connection element (3), the connector (6) being able to be fixed simultaneously to the two connection elements (2, 3).

## Patentansprüche

1. Osteosynthesesytem für das Rückgrat insbesondere für die vordere Fixierung, das folgendes enthält:
- ein längliches Verbindungsstück (2);
- eine Wirbelschraube (8), welche einen gewindeten Kopf (32) aufweist;
- ein Verbindungsstück (6) mit zwei Armen (16), welche dafür geeignet sind, auf der Schraube (8) aufgefädelt zu werden und zwischen sich das Verbindungsstück (2) einzuklemmen; und
- ein gewindetes Spannorgan (10), das dafür geeignet ist, mit dem Kopf zusammenzuwirken, um die Arme (16) festzuspannen,
**dadurch gekennzeichnet, dass**
der Kopf (32) eine gewindete Öffnung (39) aufweist, wobei das Spannorgan (10) eine gewindete Stange (42) aufweist, welche dafür geeignet ist, mit der Öffnung (39) einzugreifen.

2. System nach Anspruch 1,
**dadurch gekennzeichnet, dass**
das Spannorgan (10) eine Vertiefung mit polygonalem Profil gemäß einer Längsachse des Organs aufweist.

3. System nach einem der Ansprüche 1 oder 2,
**dadurch gekennzeichnet, dass**
das Spannorgan (10) einen Kopf (44) aufweist, der eine konvexe untere Fläche (46) aufweist, wobei einer der Arme (16) des Verbindungsstückes eine konkave kugelförmige obere Fläche (40) aufweist, die dafür geeignet ist, während dem Festspannen der Arme mit der konvexen Fläche (46) in Kontakt zu treten.

4. System nach einem der Ansprüche 1 bis 3,
**dadurch gekennzeichnet, dass**
die Öffnung (39) der Wirbelschraube (8) eine Vertiefung mit einem polygonalen Profil gemäß einer Längsachse der Schraube aufweist.

5. System nach Anspruch 4,
**dadurch gekennzeichnet, dass**
sich die Vertiefung in das Gewinde der Öffnung (39) erstreckt.

6. System nach einem der Ansprüche 2 und 4,
**dadurch gekennzeichnet, dass**
das Profil der Vertiefung der Wirbelschraube und das Profil der Vertiefung des Spannorgans (10) die gleiche Form und die gleichen Abmessungen aufweisen.

7. System nach einem der Ansprüche 1 bis 6,
**dadurch gekennzeichnet, dass**
der Kopf (32) der Wirbelschraube (8) eine glatte äußere Endseitenfläche (34) aufweist.

8. System nach einem der Ansprüche 1 bis 7,
**dadurch gekennzeichnet, dass**
es eine zweite Wirbelschraube (18) aufweist, wobei einer der Arme (16) eine Verlängerung (50) aufweist, welche dafür geeignet ist, in Eingriff auf der zweiten Schraube zu kommen.

9. System nach einem der Ansprüche 1 bis 8,
**dadurch gekennzeichnet, dass**
es ein zweites längliches Verbindungselement (3) aufweist, wobei das Verbindungsstück (6) geeignet ist, gleichzeitig an den beiden Verbindungselementen (2, 3) befestigt zu sein.
